## Europäisches Patentamt

## European Patent Office

## Office européen des brevets

(11) Veröffentlichungsnummer: **0 054 537 B1**

(12) **EUROPÄISCHE PATENTSCHRIFT**

(45) Veröffentlichungstag der Patentschrift:
26.02.86

(51) Int. Cl.⁴: **G 01 N 1/22, G 01 N 33/00**

(21) Anmeldenummer: **81890199.3**

(22) Anmeldetag: **14.12.81**

(54) **Anordnung zur Messung von flüchtigen Bestandteilen eines Kulturmediums der Fermentationsindustrie.**

(30) Priorität: **17.12.80 AT 6153/80**

(43) Veröffentlichungstag der Anmeldung:
**23.06.82 Patentblatt 82/25**

(45) Bekanntmachung des Hinweises auf die Patenterteilung:
**26.02.86 Patentblatt 86/9**

(84) Benannte Vertragsstaaten:
**CH DE FR GB LI NL**

(56) Entgegenhaltungen:
**CH - A - 457 912
DE - A - 2 310 264
FR - A - 1 573 147
FR - A - 2 247 140
GB - A - 1 452 574
US - A - 3 024 660**

**Die Akte enthält technische Angaben, die nach dem Eingang der Anmeldung eingereicht wurden und die nicht in dieser Patentschrift enthalten sind.**

(73) Patentinhaber: **Vogelbusch Gesellschaft m.b.H., Mautner Markhof Gasse 40, A-1110 Wien (AT)**

(72) Erfinder: **Die Erfinder haben auf ihre Nennung verzichtet**

(74) Vertreter: **Itze, Peter, Dipl.-Ing. et al, Patentanwälte Dipl.-Ing. Leopold Friebel Dipl.-Ing. Peter Itze Zieglergasse 57/11, A-1070 Wien (AT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf eine Anordnung zur Messung von flüchtigen Bestandteilen eines Kulturmediums der Fermentationsindustrie, mit einer Sonde, die eine Permeationsmembran zum Übertragen der zu messenden Substanzen in das Trägermedium aufweist, und einem Sensor, der auf den Gehalt der in das Trägermedium übertragenen und zu messenden Substanzen anspricht.

Bei einer bekannten Ausbildung dieser Art (DE-A-2310264) ist eine Dialysemembran an einem starren Träger vorgesehen, in welchem Kanäle zum Vorbeileiten eines Trägermediums an der Innenseite der Dialysemembran vorgesehen sind. Das mit den eindialysierten Substanzen beladene Trägermedium wird aus der Probenentnahmevorrichtung herausgeleitet und einem Analysiergerät zugeführt. Es wurde nämlich bisher angenommen, daß ein auf flüchtige Substanzen ansprechender Sensor nicht direkt in einem in den das Kulturmedium enthaltenden Raum ragenden Teil eines Sondenkörpers angeordnet werden kann, da derartige Sensoren sehr empfindlich gegenüber Wärme und vor allem gegen Feuchtigkeit sind. Weiters wurde als Voraussetzung angesehen, daß derartige Sensoren in speziellen Sensoraufnahmegefäßen angeordnet Werden müssen, um eine genaue Funktion zu erreichen.

Die bekannte Ausbildung hat nun den Nachteil, daß das Trägermedium auf seinem Weg von der Sonde zum Sensor Temperaturschwankungen unterworfen ist, wodurch Meßfehler entstehen. In der Regel kommt es dabei zu einer Abkülung, da die Temperatur des Kulturmediums höher ist als die Temperatur des Raumes, in welchem sich der das Kulturmedium enthaltende Fermenter befindet. Wenn dabei ein gasförmiges Trägermedium eingesetzt wird, kommt es bei einer Abkühlung zur Kondensation von höhersiedenden Bestandteilen des zu analysierenden Gases, so daß die Meßwerte nicht reproduzierbar sind. Außerdem können die Meßwerte durch etwaige Außeneinflüsse, wie Zugluft, Sonneneinstrahlung od.dgl., verfälscht werden.

Der Erfindung liegt die Aufgabe zugrunde, eine Anordnung der eingangs genannten Art zu schaffen, mit welcher die genannten Nachteile vermieden werden.

Erfindungsgemäß wird dies dadurch erreicht, daß der Sensor in dem Teil des Sondenkörpers angeordnet ist, der in den das Kulturmedium enthaltenden Raum ragt, daß der Innenraum des genannten Teils des Sondenkörpers zur Ableitung des Trägermediums dient, das mit den zu messenden flüchtigen Bestandteilen angereichert ist, und daß am Sondenkörper ein Kern befestigt ist, um den die Permeationsmembran angeordnet ist und der Kanäle für die Zuführung und Ableitung des Trägermediums aufweist.

Dadurch gelangt das mit den übertragenen flüchtigen Bestandteilen angereicherte Trägermedium nach Passieren der Permeationsmembran ohne einer Temperaturschwankung ausgesetzt zu sein zum Sensor, wodurch eine genaue, reproduzierbare und die tatsächlichen Verhältnisse unverfälscht wiedergebende Messung erzielt wird.

Vorteilhafterweise kann der Sensor direkt gegenüber der Ausmündung einer vom Kern in den Sondenkörper führenden Ableitung für das von der Permeationsmembran kommende, mit den zu messenden flüchtigen Besatndteilen angereicherte Trägermedium angeordnet sein. Dadurch gelangt das mit den flüchtigen Bestandteilen angereicherte Trägermedium sogleich nach Passieren der Permeationsmembran zum Sensor, so daß es zu keinerlei Reaktionen zwischen Trägermedium und flüchtigen Bestandteilen kommen kann, bevor letztere vom Sensor gemessen sind.

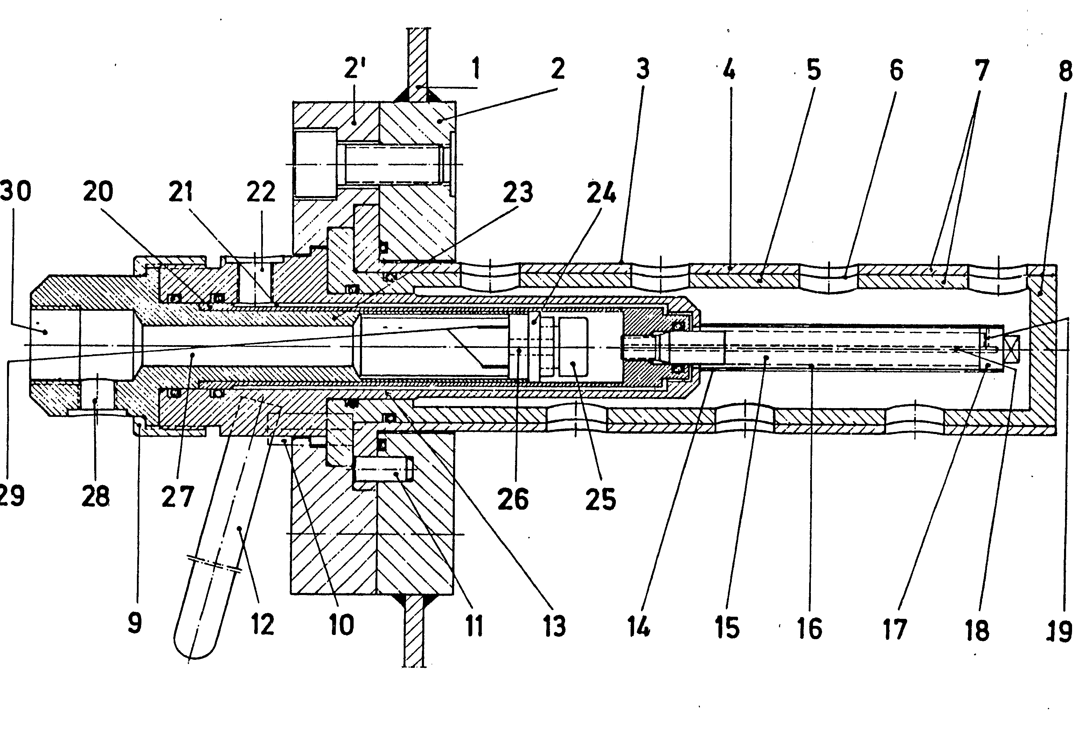

In der Zeichnung ist ein Ausführungsbeispiel des Erfindungsgegenstandes im Längsschnitt dargestellt.

Mit 1 ist die Wandung eines Fermentationsbehälters bezeichnet, in welcher ein Flansch vorgesehen ist, der aus zwei gegeneinande verschraubbaren Teilen 2 und 2' besteht. Mittels des Flansches ist an der Wandung 1 eine Schleuse 3 dicht befestigt, welche aus zwei dichtend ineinandergeschobenen, in das Behälterinnere ragenden, gelochten Rohren 4 und 5 besteht. Die beiden Rohre sind dabei gegeneinander verdrehbar und zwar derart, daß in einer Endstellung die Löcher 6 der Rohre übereinanderliegen und in der anderen Endstellung jedes Loch 6 des einen Rohres einem geschlossenen Wandungsteil 7 des anderen Rohres gegenüberliegt. Das innere Rohr 5 ist dabei an seinem behälterseitigen Ende mit einer Platte 8 verschlossen, so daß nach Verdrehen des inneren Rohres 5 in seine andere Endstellung der Innenraum dieses Rohres gegenüber dem Behälterinnenraum abgeschlossen ist. In dieser gegenseitigen Drehstellung der Rohre 4 und 5 der Schleuse 3 wird in letztere die erfindungsgemäße Sonde 9 eingefürt, wobei diese im Teil 2' des Flansches 2, 2' über einen sogenannten Bajonettverschluß befestigt ist. An der Sonde 9 ist ein Stift 10 angeordnet, welcher in eine entsprechende Bohrung des inneren Rohres 5 eingreift, wodurch beim Verdrehen der Sonde zwecks Schließens des Majonettverschlusses das innere Rohr 5 mit der Sonde 9 mitgedreht wird. Das äußere Rohr 4 ist dabei mittels eines in eine Bohrung des Teiles 2 des Flansches 2, 2' eingreifenden Stiftes 11 gegen Verdrehen gesichert. Zum Verdrehen der Sonde 9 ist diese mit einem Handgriff 12 versehen.

Die Sonde 9 weist einen Sondenkörper 13 auf, an dem der Stift 10 und der Bajonettverschluß angeordnet sind. Am Sondenkörper 13 ist an seinem in das Behälterinnere ragenden Ende eine Permeationsmembran 14 vorgesehen, welche durch einen Silikonschlauch gebildet ist und auf einen am Sondenkörper 13 befestigten zylindrischen Kern 15 aufgeschoben ist, der an seiner Mantelaußenseite ein Gewinde aufweist, dessen Gänge durch den die Permeationsmembran 14 bildenden Silikonschlauch abgeschlossen sind. Dadurch ergeben sind, je nach Gängigkeit des Gewindes, zwischen der Mantelaußenseite des Kerns 15 und der Permeationsmembran ein oder mehrere schraubenlinienförmig verlaufende Kanäle 16, durch welche im Betrieb das Trägermedium strömt. An dem freien Ende des Kerns 15 ist ein gewindefreier Bereich 17 belassen, um einen Abschluß der Kanäle 16 gegenüber dem Behälterinhalt zu erreichen. Der Kern 15 weist eine in seiner Längsrichtung verlaufende Sackbohrung 18 auf, welche nahezu den gesamten kern durchsetzt und erst im gewindefreien Bereich 17 endet. In diesem Bereich ist eine Bohrung 19 vorgesehen, durch welche das Sackloch 18 mit den durch die Gewindegänge gebildeten Kanälen 16 verbunden ist. Der Sondenkörper 13 ist hohl ausgebildet, wobei in den betreffenden Hohlraum ein rohrförmiger Teil 20 eingeschoben ist, und zwar unter Freilassung eines Zwischenraumes zwischen den Wandungen, wodurch ein Kanal 21 entsteht, welcher mit dem sondenkörperseitigen Ende der Kanäle 16 verbunden ist und einen nach außen führenden Anschlußstutzen 22 für die Zuleitung des Trägermediums aufweist.

In diesen rohrförmigen Teil 20 ist ein Sensorträger 23 koaxial eingeschoben, welcher an seinem zum Kern 15 hin weisenden Ende einen Sockel 24 für den Sensor 25 aufweist, wodurch letzterer direkt gegenüber der Ausmündung des das Trägermedium aus dem Kern 15 herausführenden Sackloches 18 angeordnet ist. Das durch den Sensor 25 hindurchgehende, mit den flüchtigen Bestandteilen angereicherte Trägermedium strömt aus der Sonde 9 durch eine Bohrung 26 im Sockel 24, eine den Sensorträger 23 in axialer Richtung durchsetzende Bohrung 27 und einen seitlichen Ableitungsstutzen 28 ab. Mit 29 sind die elektrischen Anschlüsse am Sockel 24 bezeichnet. Die nicht dargestellten Meßleitungen sind durch die Bohrung 27 aus der Sonde 9 herausgeführt, wobei die Bohrung an ihrem aus dem Behälter herausragenden Ende mittels eines Stopfens 30 abgeschlossen ist, welcher Durchführungen für die Meßleitungen aufweisen kann. Über diese Meßleitungen kann die Sonde an einen Mikroprozessor angeschlossen sein, welcher dann in Abhängigkeit von den vom Sensor kommenden Meßweren, z.B. den Zulauf der Nährlösung, steuert.

Beim Einführen der Sonde 9 in die Schleuse 3 befinden sich beide Rohre 4,5 der Schleuse in jener gegenseitigen Drehstellung, in welcher die Löcher 6 der Rohre 4, 5 durch die Wandungsteile 7 des jeweils anderen Rohres verschlossen sind. Wenn die Sonde 9 nun ganz eingeführt ist, dann greift der Stift 10 der Sonde in eine entsprechende Bohrung des inneren Rohres 5. Wird nun die Sonde 9 mittels des Bajonettverschlusses durch Verdrehen verankert, dann wird gleichzeitig das innere Rohr 5 in seine andere Endstellung gedreht, in welcher die Löcher 6 der Rohre 4, 5 einander gegenüberliegen, wodurch der Schleuseninnenraum von Kulturmedium durchströmt werden kann und solcherart die Permeationsmembran 14 mit dem Kulturmedium in Kontakt kommt. Es wird nun über den Anschlußstutzen 22 und den Kanal 21 Trägermedium, vorzugswelse Preßluft, in die durch das Gewinde gebildeten Kanäle 16 gefördert, wo die zu messenden flüchtigen Bestandteile durch die Permeationsmembran 14 aus dem Kulturmedium in das Trägermedium einpermeiren, also auf das Trägermedium übertragen werden. Das Trägermedium gelangt dann am vorderen Ende des die Permeationsmembran 14 tragenden Kernes 15 über die Bohrung 19 in das in Axialrichtung des Kernes 15 verlaufende Sackloch 18 und strömt unmittelbar nach Verlassen dieses Sackloches direkt auf den Sensor 25, durchströmt diesen und verläßt die Sonde 9 über die Bohrung 26 im Sockel 24 des Sensors, die Bohrung 27 im Sensorträger 23 und den Abflußstutzen 29. Der Sensor 25 reagiert dabei auf den Gehalt an flüchtigen Substanzen mit einer Änderung des Leitungswiderstandes und liefert solcherart ein Meßsignal für den Mikroprozessor, der dann in Abhängigkeit von diesem Meßsignal die nötigen Steuerungsmaßnahmen durchführt.

Soll die Sonde 9, etwa zwecks Reinigung od. dgl., aus der Schleuse 3 herausgezogen werden, dann wird sie mittels des Handgriffes 12 zwecks Lösens des Bajonettverschlusses verdreht, wodurch gleichzeitig über den Stift 10 das innere Rohr 5 der Schleuse 3 so weit verdreht wird, daß die Löcher 6 der Rohre 4,5 von den geschlossenen Wandungsteilen 7 des jeweils anderen Rohres abgedeckt werden, wodurch die Schleuse 3 geschlossen ist.

**Patentansprüche**

1. Anordnung zur Messung von flüchtigen Bestandteilen eines Kulturmediums der Fermentationsindustrie, mit einer Sonde (9) die eine Permeationsmembran (14) zum Übertragen der zu messenden Substanzen in das Trägermedium aufweist, und einem Sensor (25), der auf den Gehalt der in das Trägermedium übertragenen und zu messenden Substanzen anspricht, dadurch gekennzeichnet, daß der Sensor (25) in dem Teil des Sondenkörpers (13) angeordnet ist, der in den das Kulturmedium enthaltenden Raum ragt, daß der Innenraum des genannten Teils des Sondenkörpers zur Ableitung des Trägermediums dient, das mit den zu messenden flÜchtigen Bestandteilen angereichert ist, und daß am Sondenkörper (13) ein Kern (15) befestigt ist, um den die Permeationsmembran (14) angeordnet ist und der Kanäle (16,18,19) für die Zuführung und Ableitung des Trägermediums aufweist.

2. Anordnung nach Anspruch 1, dadurch gekennzeichnet, daß der Sensor (25) direkt gegenüber der Ausmündung einer vom Kern (15) in den Sondenkörper (13) führenden Ableitung (18) für das von der Permeationsmembran (14) kommende, mit den zu messenden flüchtigen Bestandteilen angereicherte Trägermedium angeordnet ist.

## Claims

1. Apparatus for measuring volatile components in a culture medium in the fermentation industry, having a probe (9) which comprises a permeation membrane (14) for tranferring the substances to be measured into the carrier medium, and a sensor (25) which responds to the proportion of substances to be measured which have been transferred to the carrier medium, characterised in that the sensor (25) is arranged in the part of the probe body (13) which projects into the space containing the culture medium, the interior space of the said part of the probe body serves to draw off the carrier medium enriched with the volatile components to be measured, and secured to the probe bode (13) is a core (15) around which the permeation membrane (14) is arranged and which comprises ducts (16, 18, 19) for the supply and drawing off of the carrier medium.

2. Apparatus according to claim 1, characterised in that the sensor (25) is arranged directly opposite the mouth of an outlet (18) leading from the core (15) into the probe body (13), for the carrier medium which comes from the permeation membrane (14) and which is enriched with the volatile components to be measured.

## Revendications

1. Dispositif de mesure de composés volatils d'un milieu de culture dans l'industrie de la fermentation, avec une sonde (9) qui présente une membrane de perméation (14) pour le transfert des substances à mesurer dans le milieu porteur, et un palpeur (25) qui répond à la teneur des substances à mesurer qui ont été transférées dans le milieu porteur, caractérisé en ce que le palpeur (25) est disposé dans la partie du corps (13) de la sonde qui plonge dans la chambre contenant le milieu de culture, que l'espace intérieur de la partie mentionnée du corps de la sonde sert à l'évacuation du milieu porteur qui est enrichi des composés volatils à mesurer et ou'au corp (13) de la sonde est fixé un noyau (15) autour duquel est disposée la membrane de perméation (14) et qui présente des canaux (16, 18, 19) pour l'entrée et la sortie du milieu porteur.

2. Dispositif suivant la revendication I, caractérisé en ce que le palpeur (25) est disposé directement vis à vis de l'orifice de sortie d'une conduite d'évacuation (18), allant du noyau (15) au corps (13) de la sonde, pour le milieu porteur enrichi des composés volatils à mesurer qui provient de la membrane de perméation (14).

0 054 537